# EUROPEAN PATENT APPLICATION

(11) **EP 4 130 713 A1**
(43) Date of publication of application: **08.02.2023**
(21) Application number: 21776510.6
(22) Date of filing: 24.02.2021
(51) Int. Cl.: G01N 15/14, C12M 1/34, C12Q 1/06, G01N 33/483, G01N 21/49, G01N 21/53

(54) **IMAGING FLOW CYTOMETER, SORTING METHOD, AND CALIBRATION METHOD**

(30) Priority: 24.03.2020 JP 2020052197; 29.07.2020 JP 2020128502
(71) Applicant: K.K. Cybo, Tokyo 135-0064 (JP)
(72) Inventor: NITTA, Nao, Tokyo 135-0064 (JP); SUGIMURA, Takeaki, Tokyo 135-0064 (JP)
(74) Representative: Global IP Europe Patentanwaltskanzlei
(86) International application number: PCT/JP2021/006818
(87) International publication number: WO 2021/192786

(57) **Abstract**

To provide an imaging flow cytometer with high sort accuracy adjusted for velocity variations among objects with a simplified configuration. An imaging flow cytometer 1 includes: a laser unit 20 that emits first and second laser light to first and second spots 25a, 25b; a first and second imaging sections 52a, 52b that image the first and second spots 25a, 25b; first and second detection devices 32a, 32b that detect a particle P that passes through the first and second spots 25a, 25b; a first particle detection section 70 that issues an imaging timing instruction signal SG1 to the first and second imaging sections 52a, 52b; an image storage section 82 that receives an image D1 imaged by the first and second imaging sections 52a, 52b; and a sorting determination section 84 that determines whether the particle P is an objective particle. The first and second imaging sections 52a, 52b clip an image D1 of the particle P based on the imaging timing instruction signal SG1.

## Description

### FIELD

The present invention relates to an imaging flow cytometer, a sorting method, and a calibration method for sorting objects such as cells using images.

### BACKGROUND

In general, a flow cytometer that sorts objects such as cells includes: a detection section that detects a signal derived from an object; a sorting determination section that determines whether the object is a specific object based on the signal obtained by the detection section; and a sorting section that sorts the object based on the determination of the sorting determination section (e.g., Patent Literature 1). To improve the accuracy of sorting objects, the sorting section must operate in step with the timing at which a specific object arrives at the sorting section.

In processing a signal, the sorting determination section of conventional flow cytometers requires just a short time of about one millisecond or less. Practically, the velocity of objects flowing through the channel varies depending on the objects. For example, cells of different sizes may have different velocities. Even among cells of the same size, those flowing nearer to the center of the channel will have a higher velocity.

Here, the effect of the variations in velocity among the objects can be suppressed by shortening the distance from the detection section to the sorting section. Accordingly, the conventional flow cytometers generally do not consider the variations in velocity among the objects and have their sorting section operate at a certain delay time after an object passes through the detection section.

On the other hand, imaging flow cytometers that use images to determine whether an object is a specific object can take longer signal processing time to obtain the necessary information, for example, about 2 to 32 milliseconds. In this case, the distance from the detection section to the sorting section must be increased. If the sorting section is set to operate at a certain delay time after an object passes through the detection section without considering the variations in velocity among the objects, the velocity variations reduce the sorting accuracy.

In order to suppress the reduction in sorting accuracy due to such velocity variations, there have been proposed configurations including a velocity measurement section that measures the velocity of each object. For example, Patent Literature 1 discloses a velocity measurement section that uses a grating (a diffraction grating). Non Patent Literature 1 discloses a configuration including laser spots set upstream and downstream of a channel through which objects flows. The velocity of each object is measured by the timings at which the object passes through the laser spots.

Patent Literature 2 also discloses a method including: capturing time-lapse images of an object; calculating the velocity of the object from changes in position of the object among frames; and estimating the arrival timing at the sorting section for each of the objects.

### CITATION LIST

### PATENT LITERATURE

Patent Literature 1: US 6,532,061 B
Patent Literature 2: WO 2011/105507 A

### NON PATENT LITERATURE

Non Patent Literature 1: N. Nitta et al., Intelligent Image-Activated Cell Sorting. Cell 175, 266-276 (2018)

### BRIEF SUMMARY

### TECHNICAL PROBLEM

The velocity measurement section that uses the grating as in Patent Literature 1 will have difficulty in measuring the velocity of each of a plurality of objects that flow through a channel closely to each other.

Even when the velocity measurement section is capable of measuring the velocity of each object, in Patent Literature 1, a velocity signal and cell classification are calculated independently of each other and the results are input to a delay processing circuit to control the sorting section. Therefore, there is no assurance that each image captured by a camera and the velocity of each object measured by the velocity measurement section are accurately associated with each other in a one-to-one correspondence. If the image processing result and the velocity information derived from different objects are incorrectly associated with each other, the sorting fails disadvantageously. Specifically, when the objects are fed to the channel at high throughput, the signal interval between the objects becomes so narrow that signals may overlap and become buried, causing errors in associating. When measured objects are small in size, an object may be successfully identified by the camera but not by the velocity detection section. In this case also, an error may occur in establishing a one-to-one correspondence of signals.

The configuration in Non Patent Literature 1 that uses two laser spots is capable of measuring the velocity of each of a plurality of objects close to each other. However, the configuration with two laser spots further requires laser emissions to two locations for velocity detection separately from the laser emission for cell imaging, which makes the apparatus large in scale. Furthermore, the timings of issuing the signals for velocity detection and cell imaging depend on the positions of the laser spots. Therefore, unless the positions of the laser spots perfectly match, the timings will not be identical. That is, the configuration necessitates calibration to compensate for the timing gap and complicates the operations.

What is normally used in imaging and detecting particles flowing in a flow cytometer is an objective lens, whose field of view is limited. The method of Non Patent Literature 1 requires at least two spots for velocity measurement and one spot for imaging to be located within the field of view of the objective lens. These spots must have a certain amount of space between each other in order to avoid stray light and others. Therefore, the allocation of laser spots in the field of view of the objective lens becomes a challenge, particularly when a plurality of spots are to be set for imaging. For example, when it is desired to capture fluorescent images separately for excitation light of different wavelengths, the laser spots may be set at different positions for the excitation light of different wavelengths, respectively. However, adding another two spots for velocity detection would be a design burden.

Patent Literature 2 discloses a reliable cell sorting method including: capturing time-lapse images of cells flowing through a channel by an imaging camera; and tracking the cells according to the series of images. Here, the method of Patent Literature 2 incurs a large computational load by tracking cells according to the series of time-lapse images. In addition, the tracking cells requires imaging with a wide field of view covering a cell selecting section. Thus, cell images with high spatial resolution can only be obtained with a screen of an extremely high pixel count, which entails a great information processing load. On the other hand, reducing the pixel count sacrifices spatial resolution, which limits the ability to obtain detailed morphological information of cells.

An object of the present invention is to provide an imaging flow cytometer, a sorting method, and a calibration method with high sort accuracy adjusted for velocity variations among objects with a simplified configuration.

### SOLUTION TO PROBLEM

A first aspect of the present invention is an imaging flow cytometer including:
a laser unit that emits first laser light and second laser light respectively to a first spot and a second spot on a channel in which a particle flows;
a first imaging section that images the first spot;
a second imaging section that images the second spot;
a first detection device that detects the particle that passes through the first spot;
a second detection device that detects the particle that passes through the second spot;
a first particle detection section that detects the particle based on signals respectively acquired by the first detection device and the second detection device, the first particle detection section issuing, to the first imaging section and the second imaging section, an imaging timing instruction signal that instructs an imaging timing;
a system time management section that issues system times to the first particle detection section;
an image storage section that receives images respectively imaged by the first imaging section and the second imaging section; and
a sorting determination section that determines whether the particle is an objective particle, wherein
the first imaging section and the second imaging section each clip out the image of the particle based on the imaging timing instruction signal.

A second aspect of the present invention is a sorting method including:
emitting, by a laser unit, first laser light and second laser light respectively to a first spot and a second spot on a channel in which a particle flows;
detecting, by a first detection device, the particle that passes through the first spot;
detecting, by a second detection device, the particle that passes through the second spot;
imaging the first spot by a first imaging section and imaging the second spot by a second imaging section and clipping out an image of the particle by each of the first imaging section and the second imaging section based on an imaging timing at which the first detection device and the second detection device acquire signals;
determining, by a sorting determination section, whether the particle is an objective particle;
calculating a delay time to an arrival of the particle at a sorting section based on a timing at which the first detection device and the second detection device acquire a signal; and
sorting, by the sorting section, the objective particle based on the delay time of the particle according to the determination of the sorting determination section.

A third aspect of the present invention is a calibration method including:
emitting, by a laser unit, first laser light and second laser light respectively to a first spot and a second spot on a channel in which a particle flows;
detecting, by a first detection device, the particle that passes through the first spot;
detecting, by a second detection device, the particle that passes through the second spot;
calculating an estimated arrival time of the particle at which the particle passes through a detection position positioned downstream from the first spot and the second spot on the channel based on timings at which the first detection device and the second detection device acquire signals;
detecting the particle that passes through the detection position and detecting an arrival time at which the particle arrives at the detection position, and
adjusting the estimated arrival time based on the arrival time.

### ADVANTAGEOUS EFFECTS

The present invention provides an imaging flow cytometer, a sorting method, and a calibration method with high sort accuracy adjusted for velocity variations among objects with a simplified configuration.

### BRIEF DESCRIPTION OF DRAWINGS

FIG. 1 is a configuration diagram of an imaging flow cytometer according to a first embodiment.
FIG. 2A is an enlarged view of a channel around spots.
FIG. 2B is an intensity profile of laser light.
FIG. 3 is a configuration diagram of an imaging section.

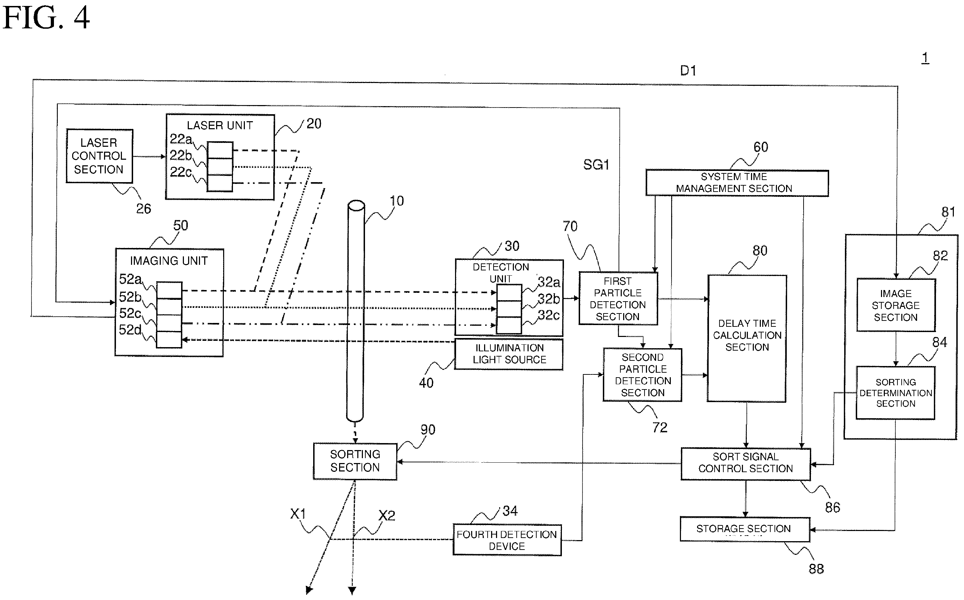

FIG. 4 is a configuration diagram of an imaging flow cytometer according to a second embodiment.
FIG. 5 is a graph of an exemplary calibration method.

### DETAILED DESCRIPTION

### First Embodiment

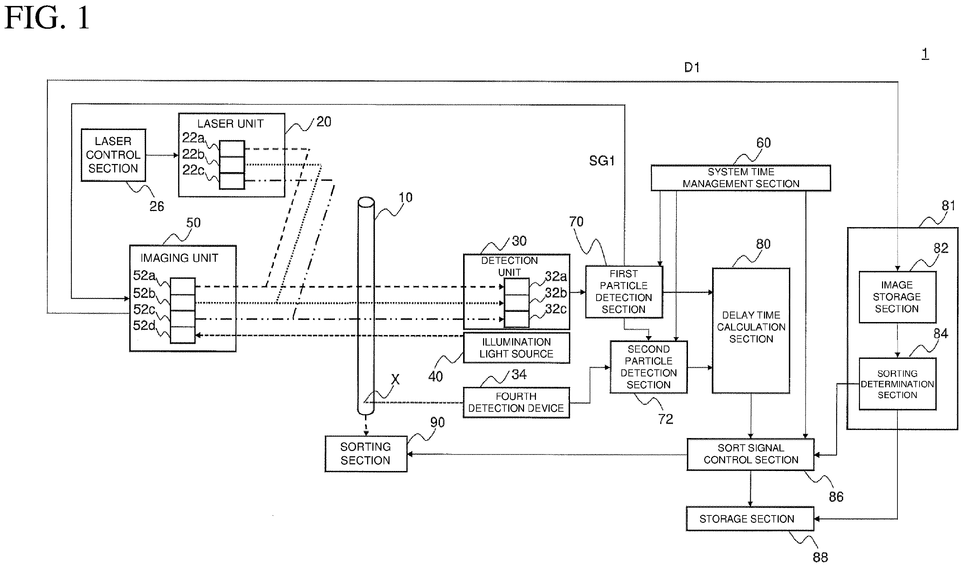

In the following, with reference to the drawings, a description will be given of an imaging flow cytometer 1 according to a first embodiment. FIG. 1 is a configuration diagram of the imaging flow cytometer 1 according to the present embodiment. The imaging flow cytometer 1 according to the present embodiment determines whether particles P flowing in the channel 10 are objective particles to be sorted and sorts the objective particles. The particles P are, for example, beads, cells and cell clusters (blood cells, bone marrow cells, lymphocytes, circulating cancer cells, vascular endothelial cells, platelets, platelet aggregates, eggs, sperm, fertilized eggs, spheroids, organoids and others), organelles (chromosomes, chloroplasts, mitochondria and others), microbes, parasites, pollen, algae (Chlamydomonas, Euglena and others) and others. A large number of particles P flow in a certain direction (axial direction) through the channel 10.

The imaging flow cytometer 1 mainly includes: a laser unit 20, a detection unit 30, a first particle detection section 70, an imaging unit 50, a system time management section 60, and an image analysis section 81.

The laser unit 20 includes at least two laser light sources. The laser unit 20 according to the present embodiment includes a first laser light source 22a, a second laser light source 22b, and a third laser light source 22c. The laser light sources 22a, 22b, 22c preferably emit laser light of different wavelengths.

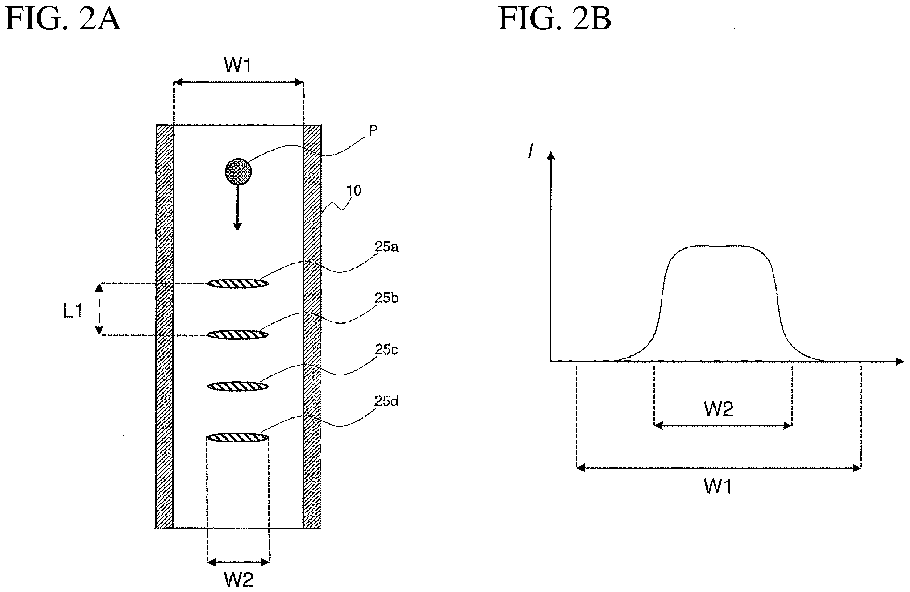

The first laser light source 22a irradiates a first spot 25a on the channel 10 with first laser light. The second laser light source 22b irradiates a second spot 25b on the channel 10 with second laser light. The third laser light source 22c irradiates a third spot 25c on the channel 10 with third laser light. FIG. 2A is an enlarged view of the channel 10 around the spots.

Here, an inner diameter W1 of the channel 10 is about 200 µm. The spots 25a to 25c extend in the direction perpendicular to the axis of the channel 10. The width of the spots 25a to 25c in the direction perpendicular to the channel 10 is, for example, 80 µm to 100 µm. The diameter of each particle P is, for example, 20 µm.

The spots 25a to 25c are spaced apart from each other in the axial direction of the channel 10. The spots 25a to 25c may or may not be disposed at regular intervals. Placement of the spots 25a to 25c will be described later.

FIG. 2B shows an exemplary intensity profile of laser light. The laser light preferably has a constant intensity in the direction perpendicular to the axis of the channel 10.

The detection unit 30 includes at least two detection devices. The detection unit 30 according to the present embodiment includes a first detection device 32a, a second detection device 32b, and a third detection device 32c. The first detection device 32a detects scattered light that occurs when a particle P passes through the first spot 25a. Similarly, the second and third detection devices 32b, 32c detect scattered light that occurs when the particle P passes through the second and third spots 25b, 25c, respectively.

Here, a distance L1 between the first spot 25a and the second spot 25b is determined by the distance between a line sensor 100 on a first imaging section 52a and a line sensor 100 on a second imaging section 52b, and the optical magnification of an image forming optical system which will be described later. That is, the distance L1 is calculated from such design values. Therefore, from the timing information of a particle P passing through the first spot 25a and the second spot 25b, the velocity for the particle P is calculated on a particle-by-particle basis.

Based on the scattered light detected by the detection unit 30, the first particle detection section 70 detects the timings at which a particle P passes through the spots 25a to 25c. Here, as will be described later, system times are issued from the system time management section 60 to the first particle detection section 70. The first particle detection section 70 acquires a system time for each timing at which a particle P passes through the spots 25a to 25c.

The first particle detection section 70 provides an identification number to the detected particle P on a particle-by-particle basis. Furthermore, the first particle detection section 70 outputs, to a delay time calculation section 80 which will be described later, the system time for each timing at which the particle P passes through the spots 25a to 25c together with the identification number given to the particle P.

The first particle detection section 70 transmits, to the imaging unit 50, an imaging timing instruction signal SG1 with the identification number of each particle P.

The imaging unit 50 includes at least two imaging sections. The imaging unit 50 according to the present embodiment includes a first imaging section 52a, a second imaging section 52b, a third imaging section 52c, and a fourth imaging section 52d. The first imaging section 52a images the first spot 25a. Similarly, the second and third spots 52b, 52c image the second and third spots 25b, 25c, respectively. The fourth imaging section 52d will be described later.

Between the first to fourth imaging sections 52a to 52d and the first to fourth spots 25a to 25d on the channel 10, the image forming optical system including a lens is disposed. The image forming optical system is disposed to form images on the first to fourth spots 25a to 25d on the line sensors 100 on the first to fourth imaging sections 52a to 52d, respectively. Here, the width of the spots 25a to 25d is, for example, 80 µm to 100 µm. The width of the line sensors 100 on the first to fourth imaging sections 52a to 52d is, for example, 10 mm to 80 mm. The magnification of the image forming optical system is, for example, 100x to 1000x.

Based on the imaging timing instruction signal SG1 from the first particle detection section 70, each of the imaging sections 52a to 52d clip out an image D1 of the particle P. Furthermore, each of the imaging sections 52a to 52d transmit, to an image storage section 82 which will be described later, the clipped image D1 of the particle P and the identification number of the particle P.

Here, a description will be given of placement of the spots 25a to 25c. The first to third imaging sections 52a to 52c, the image forming optical system, and the channel 10 are preferably fixed so that images of a particle P flowing through the channel 10 are formed at the line sensors 100 (described later) on the first to third imaging sections 52a to 52c. In this case, the positions of the spots 25a to 25c are fine-tuned so that the images of the particle P passing through the spots 25a to 25c are formed at the line sensors 100 on the first to third imaging sections 52a to 52c, respectively. Thus, the distances among the spots 25a to 25c are determined by the distances among the line sensors 100 on the first to third imaging sections 52a to 52c, respectively, and the optical magnification of the image forming optical system. Then, from the measured timing difference as to a particle P in the channel 10 passing through the spots 25a to 25c, the traveling velocity can be calculated for the particle P. Conversely, when the traveling velocity of a particle P flowing in the channel 10 is known, the timing difference as to the particle P passing through the spots 25a to 25c can be calculated.

For example, mirrors whose angle to the first to third laser light is adjustable are disposed between the laser unit 22 and the channel 10. The positions of the spots 25a to 25c can be adjusted by adjusting the angle of the mirrors.

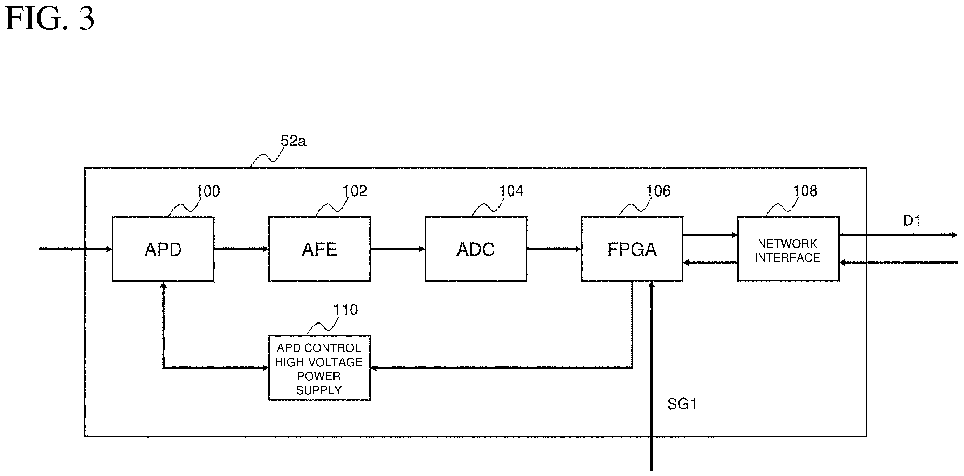

The first to fourth imaging sections 52a to 52d are similarly configured. In the following, with reference to FIG. 3, a description will be given of the configuration of the imaging sections. FIG. 3 shows an exemplary configuration of the first imaging section 52a. The first imaging section 52a includes a line sensor 100. The line sensor 100 is, for example, an avalanche photodiode array (APD array), a photodiode array (PD array), or a photomultiplier tube array (PMT array). Corresponding to a received image, each pixel of the line sensor 100 outputs a signal current or a signal voltage. While these outputs are performed parallel across all pixels, the outputs of multiple pixels may be switched in a time-division manner and provide the outputs to the subsequent element.

The line sensor 100 in FIG. 3 is an APD array. The line sensor 100 extends in the direction in which the first spot 25a extends. When the APD array is used, an APD control high-voltage power supply 110 controls the applying voltage so that the output becomes constant against variations in gain characteristics and others due to temperature changes or the like. When any light receiving element other than the APD array is used, a control circuit that controls the bias voltage or bias current and gain of the element is connected.

The current or voltage output from the line sensor 100 is amplified by an analog front-end (AFE) circuit 102 and converted into AD-convertible voltage signal. When needed, a transimpedance amplifier (TIA) circuit that converts current to voltage and amplifies the signal may be connected between the APD array 100 and the analog front-end circuit 102. The analog front-end circuit 102 is connected in parallel to the output of all the pixels of the APD array 100. Here, the pixels may be switched in a time-division manner and provide the outputs to the subsequent element.

The analog front-end circuit 102 outputs voltage signals to the AD converter 104. While the AD converter 104 is connected to receive the outputs of all the pixels of the analog front-end circuit 102 in parallel, the outputs of the analog front-end circuit 102 may be switched in a time-division manner and provided to the subsequent AD converter 104.

When the AD converter 104 is connected to receive the outputs of all the pixels of the analog front-end circuit 102 in parallel, the sampling rate of the AD converter 104 is, for example, at least V/X × 10⁶ samples/s where V m/s is the velocity of a particle P passing through the channel, and X µm is the size of one pixel of an image in the flow direction of the particle.

The line image converted to a digital signal by the AD converter 104 is input to an FPGA 106. The FPGA 106 may be an FPGA (Field Programmable Gate Array) equipped with internal memory serving as an image buffer, a combination of the FPGA and external memory such as DRAM (Dynamic Random Access Memory), a dedicated or general-purpose logic circuit replacing the FPGA, or a computer system consisting of a microprocessor and memory.

The FPGA 106 stores data of line images of a certain time. When an imaging timing signal SG1 is received, an image acquired from the timing specified by the imaging timing signal SG1 for a preset period of time is clipped out. The clipped image is output with time information corresponding to the timing signal SG1 or the identification number of the imaged particle P. Here, the FPGA 106 may form a one-line image from line images of two or more lines by arithmetic processing such as integration to extend the bit length of the data and widen the dynamic range before output. The FPGA 106 may compress an image to reduce the data volume. In compressing data, the FPGA 106 may use variable-length coding, and redundancy in the temporal or spatial direction of the image data. The FPGA 106 may analyze the obtained image and calculate the feature values and others corresponding to the structure and shape of the cell, and output the result as information appended to the image or information independent of the image with the identification number.

The clipped image D1 or image information D1 is output from the network interface 108 as a data packet to the image storage section 82 (described later) via a network. The network interface 108 is, for example, a network interface for a different band such as 1 Gb Ethernet (GbE) or 10 Gb Ethernet. Alternatively, the clipped image D1 or image information D1 is output to the image storage section 82 by a high-speed digital interface such as PCIe or high-speed LVDS or a general-purpose interface such as a USB.

A cylindrical lens or a lens array may be disposed at the front of the line sensor 100. This improves the light collection efficiency. A bandpass filter may be disposed at the front of the line sensor 100. This improves specificity. A slit may be disposed at the front of the line sensor 100. This improves the spatial resolution in the flow direction.

The system time management section 60 consists of, for example, a system clock whose value changes every certain time, and a counter that counts the system clock. The system time management section 60 retains the system times of the whole system, and issues the system times. The system times issued by the system time management section 60 are transmitted to the first particle detection section 70, a second particle detection section 72 which will be described later, and a sort signal control section 86 which will be described later.

The image analysis section 81 includes an image storage section 82 and a sorting determination section 84. The image storage section 82 receives the image D1 captured by the imaging unit 50 together with the identification number of the particle P. The image storage section 82 may be an FPGA that includes a network interface or a general-purpose interface for receiving the image D1 and equipped with internal memory serving as an image buffer, a combination of the FPGA and external memory such as DRAM, a dedicated or general-purpose logic circuit replacing the FPGA, or a computer system consisting of a microprocessor and memory.

The sorting determination section 84 determines whether a particle P is the objective particle. Specifically, the sorting determination section 84 determines whether a particle P is the objective particle by analyzing the image of the particle P output from the image storage section 82. The sorting determination section 84 outputs the determination result together with the identification number of the particle P to a sort signal control section 86 and a storage section 88 which will be described later.

The sorting determination section 84 can use various image analysis methods according to the type of the objective particle. For example, the sorting determination section 84 can use: a method of calculating one or more feature values from the image and classifying the particle P based on the distribution of the feature values; a method of applying machine learning such as SVM (Support Vector Machine) to the distribution of the feature values to classify; and a method of applying deep learning to the image.

Alternatively, as disclosed in USP 6,211,955 B, the sorting determination section 84 may use a method of classifying based on the presence/absence of bright spots from an internal fluorescent image of a cell nucleus.

The imaging flow cytometer 1 according to the present embodiment further includes a laser control section 26, a fourth detection device 34, an illumination light source 40, a second particle detection section 72, a delay time calculation section 80, a sort signal control section 86, a storage section 88, and a sorting section 90.

The illumination light source 40 is, for example, a light emitting diode (LED). The illumination light source 40 is disposed on the side opposite to the imaging unit 50 with reference to the channel 10. The light from the illumination light source 40 becomes incident on a fourth spot 25d on the channel 10 by an illuminating optical system disposed between the illumination light source 40 and the channel 10. The illuminating optical system includes, for example, at least one lens. The illuminating optical system may be, for example, a Köhler illumination or a critical illumination. It is desired to deform the fourth spot 25d to extend in the direction perpendicular to the axis of the channel 10 by disposing a slit or a cylindrical lens on the illuminating optical system.

The fourth imaging section 52d images the fourth spot 25d. Here, the fourth imaging section 52d, the image forming optical system, and the channel 10 are preferably fixed so that an image of a particle P flowing through the channel 10 is formed at the line sensor 100 of the fourth imaging section 52d. In this case, the position of the fourth spot 25d is fine-tuned so that the image of the particle irradiated at the fourth spot 25d is formed at the fourth imaging section 52d. Adjusting in this manner, the distance between the first to third spots 25a to 25c and the fourth spot 25d is determined by the distances among the line sensors 100 on the first to third imaging sections 52a to 52d and the optical magnification of the image forming optical system. Based on the foregoing, by measuring the timing of a particle P in the channel 10 passing through the spots, the traveling velocity of the particle P can be calculated. Conversely, when the traveling velocity of a particle P flowing in the channel 10 is known, the timing difference as to the particle P passing through the spots 25a to 25d can be calculated. For example, a mirror whose angle is adjustable or an optical device such as a slit or a lens whose position is adjustable is disposed on the optical path of the illuminating optical system. The position of the fourth spot 25d can be adjusted by adjusting the angle or position of such an optical device. Alternatively, the position of the illumination light source 40 or the whole illuminating optical system can be adjusted.

The fourth detection device 34 detects a particle P that flows through a detection position X on the channel 10. The fourth detection device 34 is, for example, a camera, a stroboscopic camera, and laser and a detector. The camera and the stroboscopic camera acquire an image at the detection position X, and detect passage of the particle P from the image. As to the laser and the detector, the laser is caused to enter the detector and the detector's signal at the detection position X is monitored. From a change in the signal waveform that occurs when the particle P passes through the detection position X, the passage of the particle P is detected. The detection position X is preferably near the sorting position. The fourth detection device 34 outputs a detection signal to the second particle detection section 72.

The detection position X is positioned downstream from the first to fourth spots 25a to 25d. The detection position X preferably, but not essentially, matches with the sorting section 90. For example, the detection position X may be positioned slightly upstream or downstream from the sorting section 90.

The second particle detection section 72 detects the timing of the particle P passing through the detection position X based on the scattered light or fluorescence detected by the fourth detection device 34. The second particle detection section 72 receives the system times issued by the system time management section 60. The second particle detection section 72 stores the system time (arrival time) corresponding to the timing of the fourth detection device 34 detecting a detection signal together with the detection signal. That is, a time stamp is added to each detection signal.

The second particle detection section 72 processes in real time the input of detection signals from the fourth detection device 34 and the input of system times from the system time management section 60.

In the case where the fourth detection device 34 acquires images, the second particle detection section 72 may be an FPGA that receives the images from the camera and system times and that is equipped with internal memory serving as an image buffer, a combination of the FPGA and external memory such as DRAM, a dedicated or general-purpose logic circuit replacing the FPGA, or a computer system consisting of a microprocessor and memory. For example, when it is found that an image includes a particle P as a result of analysis of the image, the particle can be detected adopting the system time that was received when the image was acquired as the passage time of the particle P. In the case where the fourth detection device 34 acquires signals that are continuous in time direction as a detector, the second particle detection section 72 can be implemented as an ADC (Analog Digital Converter) that converts detection signals to digital signals and an FPGA equipped with internal memory serving as detection signal buffer and a processing circuit. The detection signal buffer and the processing circuit may be implemented as a combination of the FPGA and external memory such as DRAM, a dedicated or general-purpose logic circuit replacing the FPGA, or a computer system consisting of a microprocessor and memory. For example, by adopting the time during which changes in signals reached a certain threshold value or higher as the passage time of a particle P, the particle P can be detected.

The second particle detection section 72 outputs, to the delay time calculation section 80, the system time (arrival time) given to the detection signal corresponding to the passage of the particle P.

The laser control section 26 includes a control circuit and a driver circuit for controlling laser light. The laser control section 26 controls ON/OFF of the first to third laser light sources 22a to 22c of the laser unit 20 corresponding to the spots 25a to 25c used in imaging, and the intensity of the first to third laser light.

The delay time calculation section 80 calculates a delay time for the particle P to take to arrive at the sorting section 90, based on the system times at which the particle P passed through the first spot 25a and the second spot 25b detected by the first particle detection section 70. Specifically, the delay time ΔT is obtained by ΔT = (L2/L1) × (t2 - 11) where t1 is the system time at which the particle P passes through the first spot 25a, t2 is the system time at which the particle P passes through the second spot 25b, L1 is the distance between the first spot 25a and the second spot 25b, and L2 is the distance between the first spot 25a and the sorting section 90. Here, when the values L1 and L2 are known, these values may be used. As described above, L1 can be obtained from the design values. Therefore, when L2 is uncertain, the value of coefficient A = L2/L1 may be obtained by calibration.

Note that, while the delay time ΔT is the time from when the particle P passes through the first spot 25a until its arrival at the sorting section 90, the present invention is not limited thereto. The delay time ΔT may be the time from when the particle P passes through the second spot 25b until its arrival at the sorting section 90.

The delay time calculation section 80 outputs, to the sort signal control section 86, the delay time of the particle P with the identification number of the particle P.

According to the determination result of the sorting determination section 84, the sort signal control section 86 issues a sort signal required in sorting the particle P at the timing calculated based on the delay time of the particle P calculated by the delay time calculation section 80.

System times are being transmitted from the system time management section 60 to the sort signal control section 86. Accordingly, the sort signal control section 86 issues a proper sort signal to the sorting section 90 in step with the timing of the particle P arriving at the sorting section 90.

In issuing the sort signal, if the timing calculated based on the delay time is later than the system time, then the sorting at the calculated timing will be successful and the sort signal is output. If the timing calculated based on the delay time is earlier than the system time, the sorting will not be successful and the output of the sort signal may not be output. Whether the sorting is successful or not is output from the sort signal control section 86 to the storage section 88.

The sort signal is, for example, a pulse signal. The pulse signal is issued in step with the timing of the objective particle arriving at the sorting section 90, and transmitted to the sorting section 90. Alternatively, a signal in which information timing is coded may be transmitted using a separately provided signal line.

The sorting section 90 may sort objective particles by two or more types. For example, the sorting section 90 may sort particles by two types (2-way sorting) or by four types (4-way sorting). In such cases, the sorting determination section 84 further classifies the objective particle into a plurality of different types, and the classification result is included in the sort signal. Here, for example, the classification result can be included in the pulse signal using the amplitude or code of the pulse signal, for example.

Alternatively, using the pulse signal together with a separately provided signal line, the classification result may be transmitted to the sorting section 90 as a digital signal or the like.

The sort signal may further transmit a time width of continuing the sorting, in which the sort timing is the start time. In this case, the time width of continuing the sorting may be transmitted as a pulse width of the pulse signal. Alternatively, a signal having the time width of continuing the sorting coded may be transmitted using a separately provided signal line.

Note that, the system time at which the particle P arrives at the sorting section 90 may be calculated by either the delay time calculation section 80 or the sort signal control section 86.

The sort signal control section 86 may issue the sort signal only when the sorting determination section 84 determines that the particle P is the objective particle. The sort signal control section 86 may issue different sort signals between the case when the sorting determination section 84 determines that the particle P is the objective particle and when the sorting determination section 84 determines that the particle P is not the objective particle.

The storage section 88 stores the determination result on the particle P received from the sorting determination section 84 together with the identification number of the particle P. Furthermore, the storage section 88 stores, together with the determination result, the sort result of the particle P, that is, whether the sort signal control section 86 successfully issued the sort signal at the specified timing according to the determination result issued from the sorting determination section 84.

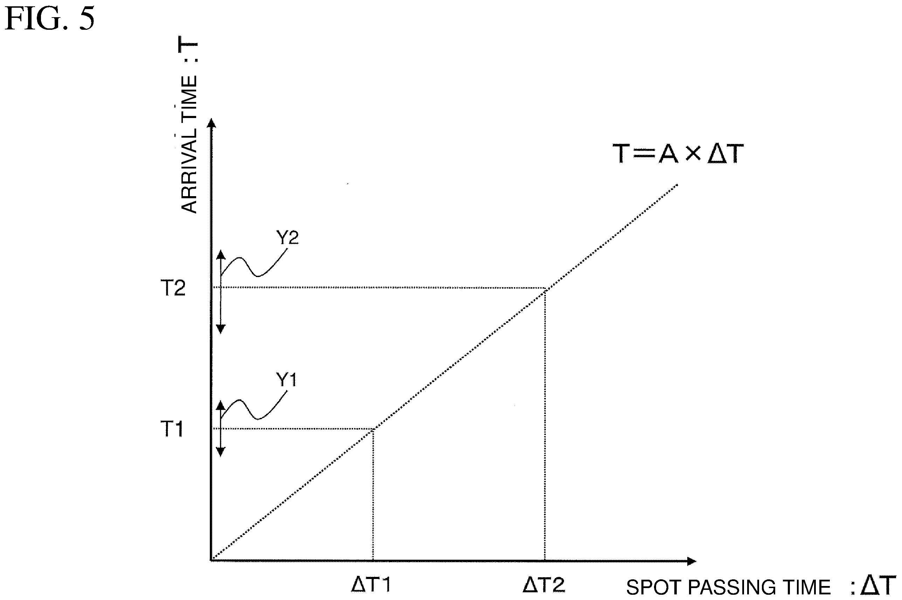

The sorting section 90 sorts the objective particle based on the sort signal issued from the sort signal control section 86. The sorting section 90 controls the sort direction switching according to information of sorting windows (W1, W2 in FIG. 5 which will be described later) received from the sort signal control section 86 or stored in the sorting section 90 based on the timing instructed by the sort signal. The sorting section 90 is, for example, a droplet sorter or an on-chip sorter.

The sorting section 90 may be, for example, the droplet sorter disclosed in M. J. Fulwyler, Science 150, 910-911 (1965). In this case, the sorting section 90 includes a nozzle disposed at the tip of the channel 10. Droplets (droplet) are formed at regular intervals at the nozzle. By applying electric field to liquid around the objective particle at the timing where a droplet including the objective particle is formed, the droplet including the objective particle is selectively charged. By providing electrostatic field around the course of the falling droplet, only the charged droplet including the objective particle selectively changes its falling course. By placing a collection container at the landing point, the objective particle is sorted out. Furthermore, by controlling the poles and amount of charges charged on the droplet thereby selectively setting the path of the droplet in different directions or angles, two or more types of cells can be separately collected into two or more individual containers.

The sorting section 90 may be, for example, an on-chip sorter disclosed in Non Patent Literature 1. In this case, a dual-membrane pump is disposed across the channel 10 to trigger a local flow perpendicularly to the channel 10 at the timing where the objective particle passes. Thus, the objective particle changes its course, and is selectively directed to a channel in a different direction at a downstream branch channel to be sorted. The dual-membrane pump is disclosed in, for example, S. Sakuma et al., Lab on a Chip 17, 2760-2767, 2017.

In the following, a description will be given of a sorting method using the imaging flow cytometer 1 according to the present embodiment. Firstly, a multitude of particles P flow through the channel 10. The laser unit 20 emits first to third laser light to the first to third spots 25a to 25c, respectively. The detection unit 30 detects each particle P passing through the first to third spots 25a to 25c. The first particle detection section 70 detects each particle P based on a signal acquired by the detection unit 30. The first particle detection section 70 issues, to the imaging unit 50, the imaging timing instruction signal SG1 instructing an imaging timing. Meanwhile, the system time management section 60 issues system times to the first particle detection section 70.

The imaging unit 50 images the first to fourth spots 25a to 25d. The imaging unit 50 clips the image D1 of an imaged particle P based on the imaging timing instruction signal SG1. The image storage section 82 receives the image D1 of the particle P imaged by the imaging unit 50. The sorting determination section 84 determines whether the particle P is the objective particle.

The sort signal control section 86 issues a sort signal in order to sort the particle P in step with the timing calculated by the delay time calculation section 80 according to the determination result of the sorting determination section 84. The sorting section 90 sorts the objective particle based on the sort signal.

The delay time calculation section 80 calculates the delay time to the arrival of the particle P at the sorting section 90 based on the system time at which the detection unit 30 acquired the signal.

In this manner, the imaging flow cytometer 1 and the sorting method according to the present embodiment improve the sort accuracy adjusting for velocity variations among the particles P with the simplified structure than the conventional technique.

### Second Embodiment

In the following, a description will be given of the imaging flow cytometer 1 according to the second embodiment. The imaging flow cytometer 1 according to the present embodiment is different from the first embodiment in the configuration of the fourth detection device 34, the second particle detection section 72, and the delay time calculation section 80. The other configurations are identical to the first embodiment. In the present embodiment, the detection position X is positioned downstream from the sorting section 90. That is, the present embodiment is capable of detecting any changes in the position of a particle P when the particle P is sorted by the sorting section 90.

The fourth detection device 34 is, for example, an area sensor such as a CCD camera or a CMOS camera. In this case, the elements are disposed so that: the fourth laser light is emitted to the detection position X; and the particle P passing through the detection position X is irradiated with the laser light emits scattered light or fluorescence, which becomes incident on the fourth detection device 34. The signal of the fourth detection device 34 is acquired to obtain the information as to the position where the particle P has passed. The fourth detection device 34 outputs the detection signal to the second particle detection section 72. The laser light can be replaced by illumination such as strobe light for detecting bright field.

The second particle detection section 72 acquires, from the position where the particle P has passed, the rate of particles that was sorted or not sorted by the sorting section, and outputs the value to the delay time calculation section 80. Here, it is not required for the fourth detection device 34 to acquire the passing position information for each particle P, and the fourth detection device 34 may acquire the sum value for a multitude of particles P. For example, when the fourth detection device 34 detects particles P having been sorted by the sorting section 90 at a position X1 in the detection position X, and detects particles P having not been sorted by the sorting section 90 at a position X2 in the detection position X, the ratio between the particles passing through the position X1 and the position X2 may be obtained as the sum of a multitude of particles. For example, when particles P can be detected by the fluorescence that is emitted by being excited by laser light, by measuring the ratio in the fluorescence intensity sum value derived from particles P at the positions X1 and X2, the rate of the particles P having been sorted and that of the particles P having not been sorted can be checked.

The delay time calculation section 80 acquires the rate of the particles P having been sorted or the particles P having not been sorted from the second particle detection section 72, to evaluate the relevance of the delay time calculation method. For example, when all the particles P are to be sorted, by adjusting the delay time to be the time by which the rate of the particles P having been sorted becomes closest to 100%, the delay time suitable to the sorting can be determined. It is also possible to calibrate the delay time calculation method by evaluating the relevance of the delay time calculation method while changing a calculation scheme for the delay time to search for the optimum delay time calculation method.

### Third embodiment

In the following, a description will be given of the imaging flow cytometer 1 according to the third embodiment. The imaging flow cytometer 1 according to the present embodiment further includes a delay time calibration function in the configuration of the imaging flow cytometer 1 according to the first embodiment. Specifically, the imaging flow cytometer 1 allows calibration-purpose beads (particles P) to flow in the channel 10, and measures the arrival time at which the particles P passing through the first spot 25a and the second spot 25b are detected at the detection position X. Thus, calibration is performed on the estimated arrival time of the particles P calculated from the particles P passing through the first spot 25a and the second spot 25b.

The imaging flow cytometer according to the present embodiment is different from the first embodiment in the configuration of the delay time calculation section 80. The other configurations are identical to the first embodiment.

The delay time calculation section 80 according to the present embodiment calculates the time at which a particle P arrives at the detection position X (estimated arrival time) based on the system times t1 and t2 at which the particle P has passed through the first spot 25a and the second spot 25b, respectively, and detected by the first particle detection section 70. Note that, the distance between the detection position X and the sorting section 90 is known.

The delay time calculation section 80 associates the identification number of a particle P and the arrival time of the particle P output from the second particle detection section 72 with each other. Specifically, the delay time calculation section 36 associates the identification number of the particle P based on the estimated arrival time of the particle P derived from the first particle detection section 70 and the arrival time of the particle P from the second particle detection section 72.

The delay time calculation section 80 outputs the associated identification number, estimated arrival time, and arrival time of the particle P as a set.

The delay time calculation section 80 performs calibration based on the estimated arrival time and arrival time of the particle P. FIG. 5 shows an exemplary calibration method. The horizontal axis indicates spot passing time ΔT calculated by the delay time calculation section 80. The spot passing time ΔT is (t2 - 11) where t1 and t2 are the times (system times) at which the particle P has passed through the first spot 25a and the second spot 25b, respectively. The vertical axis indicates arrival time T of the particle P.

When the particle P is in uniform motion, the estimated arrival time is obtained by (L2/L1) × (t2 - t1) where L1 is the distance between the first spot 25a and the second spot 25b, and L2 is the distance between the first spot 25a and the detection position X. Here, by obtaining the value of coefficient A = L2/L1 by calibration, the estimated arrival time can be obtained when t1 and t2 are given.

The delay time calculation section 80 plots the spot passing time ΔT and the arrival time T of the particle P and fits them to the model formula. The model formula is, for example, a straight line passing through the origin as in FIG. 5, but not limited thereto. The model formula is T = A × ΔT when a straight line passing through the origin is the model formula. The delay time calculation section 80 performs calibration by obtaining the coefficient A from the plots of the spot passing time ΔT and the arrival time T of the particle P.

The delay time calculation section 80 may calibrate a sorting window in addition to or in place of the estimated arrival time of the particle P. Here, the sorting window is the time period during which the sorting section 90 performs sorting. In FIG. 5, when the spot passing time of the particle P is ΔT1, the sorting is performed in the time period of a sorting window Y1 including the arrival time T1. When the spot passing time of the particle P is ΔT2, the sorting is performed in the time period of a sorting window Y2 including the arrival time T2. The width (the time length) of the sorting window may be constant irrespective of the delay time of the particle P, or may be variable depending on the delay time of the particle P. In general, when the velocity of the particle P is high, the spot passing time becomes short and the delay time becomes small. Conversely, when the velocity of the particle P is low, the spot passing time becomes long and the delay time becomes great. In FIG. 5, as the spot passing time of the particle P is longer and the delay time is greater, the width of the sorting window becomes wider. Since errors become greater as the delay time is greater, setting a wider sorting window here improves the sort accuracy.

The sorting window is not required to center the measured arrival time T. The delay time calculation section 80 can set the sorting window based on the plots of the spot passing time ΔT and the arrival time T of the particle.

The detection position X preferably, but not essentially, matches with the sorting section 90. For example, the detection position X may be positioned slightly upstream or downstream from the sorting section 90. In this case, the sorting window may be adjusted to be set at the timing where the particle P arrives at the sorting section 90, by shifting the center position of the sorting window from the estimated arrival time as appropriate by the difference in position between the detection position X and the sorting section 90. Due to the limitation on the physical operation speed of the sorting section 90, a time lag may occur between the sort signal being issued and the particle P being sorted. Assuming that the time lag is B, the sort signal generation timing may be adjusted to be earlier by the time lag B so that the sorting is performed at the timing at which the particle P arrives at the sorting section 90.

In the following, a description will be given of a calibration method and a sorting method using the imaging flow cytometer 1 according to the present embodiment.

Firstly, a multitude of particles P flow in the channel 10. The laser unit 20 emits the first to third laser light to the first to third spots 25a to 25c, respectively. The detection unit 30 detects each particle P passing through the first to third spots 25a to 25c. The first particle detection section 70 detects each particle P based on a signal acquired by the detection unit 30. The first particle detection section 70 issues, to the imaging unit 50, an imaging timing instruction signal SG1 instructing an imaging timing. Meanwhile, the system time management section 60 issues system times to the first particle detection section 70.

The second particle detection section 72 detects the timing at which the particle P passed through the detection position X, based on the signal acquired by the fourth detection device 34.

The delay time calculation section 80 calculates the estimated arrival time at which the particle P arrives at the detection position X, based on the times (system times) at which the particle P passed through the first spot 25a and the second spot 25b.

The second particle detection section 72 detects the detection signal corresponding to the passage of the particle P, and outputs the arrival time at which the particle P arrived at the detection position X to the delay time calculation section 80.

The delay time calculation section 80 performs adjustment by calibrating the formula for the estimated arrival time based on the arrival time. Then, the sort signal control section 86 and the sorting section 90 adjusts the timing of sorting the particle P based on the adjusted estimated arrival time.

Thus, the imaging flow cytometer, the sorting method, and the calibration method according to the present embodiment further improve the sort accuracy.

### Fourth Embodiment

In the following, a description will be given of the imaging flow cytometer 1 according to a fourth embodiment. The imaging flow cytometer 1 according to the present embodiment further includes a delay time calibration function in the configuration of the imaging flow cytometer 1 according to the second embodiment. Specifically, the imaging flow cytometer 1 allows calibration-purpose beads (particles P) to flow in the channel 10, and measures the arrival time at which the particles P passing through the first spot 25a and the second spot 25b are detected at the detection position X. Thus, calibration is performed on the estimated arrival time of the particles P calculated from the particles P passing through the first spot 25a and the second spot 25b.

The imaging flow cytometer according to the present embodiment is different from the third embodiment in the configuration of the fourth detection device 34, the second particle detection section 72, and the delay time calculation section 80. The other configurations are identical to the third embodiment. Additionally, the imaging flow cytometer according to the present embodiment is different from the second embodiment in the configuration of the delay time calculation section 80. The other configurations are identical to the second embodiment. In the present embodiment, the detection position X is positioned downstream from the sorting section 90. That is, the present embodiment is capable of detecting any changes in the position of a particle P when the particle P is sorted by the sorting section 90. Therefore, a sort signal is issued to the sorting section 90 at the timing at which the particle P passes through the sorting section 90. By evaluating the rate of occurrence of any positional shift due to the particle P being sorted, the timing of the sort signal issued to the sorting section 90 can be evaluated. Using this, the present embodiment performs calibration for acquiring the optimum delay time by evaluating positional changes due to sorting at the detection position X, while varying the delay time between the detection of the particle P and the issuance of the sort signal.

In the present embodiment, the second particle detection section 72 calculates the sort success rate of the particle P from the positional information of the particle P flowing in the channel 10, and outputs the determination result to the delay time calculation section 80. Note that, it is not essential for the second particle detection section 72 to receive the system time from the system time management section 60.

The delay time calculation section 80 performs calibration based on the estimated arrival time of the particle P at the sorting section 90 and the sort result determination of the particle P. The spot passing time ΔT is (t2 - 11) where t1 and t2 are times (system times) at which the particle P passed the first spot 25a and the second spot 25b, respectively. When the particle P is in uniform motion, the estimated arrival time is (L2/L1) × (t2 - t1) where L1 is the distance between the first spot 25a and the second spot 25b, and L2 is the distance between the first spot 25a and the sorting section 90. In some cases, a time lag may occur between the sort signal being issued and the particle P being sorted. Assuming that the time lag is B, the sort signal must be issued earlier just by B in order for the particle P to be properly sorted, and the optimum sort signal generation timing based on the estimated arrival time is obtained as follows: (L2/L1) × (t2 - 11) - B. L1 can be calculated from the design values. Therefore, by obtaining the values L2 and B by calibration here, the optimum delay time for a sort signal can be obtained when t1 and t2 are given. A specific calibration method may be as follows. While varying the coefficient L2 and the time lag B in a certain range, the delay time calculation section 80 instructs the sort signal control section 86 to issue a sort signal for each of particles flowing in the channel 10. The sort signal control section 86 issues signals to the sorting section 90, and the sorting section 90 attempts to sort. The second particle detection section 72 calculates the sort success rate for every coefficient L2 and time lag B, and outputs the determination result to the delay time calculation section 80. The delay time calculation section 80 performs calibration by: acquiring the relationship between the coefficient L2 and the time lag B and the sort success rate; and deriving the coefficient L2 and the time lag B which provide the maximum sort success rate.

### Reference Signs Lists

1 imaging flow cytometer
10 channel
20 laser unit
22a first laser light source
22b second laser light source
22c third laser light source
25a first spot
25b second spot
25c third spot
25d fourth spot
26 laser control section
30 detection unit
32a first detection device
32b second detection device
32c third detection device
34 fourth detection device
40 illumination light source
50 imaging unit
52a first imaging section
52b second imaging section
52c third imaging section
52d fourth imaging section
60 system time management section
70 first particle detection section
72 second particle detection section
80 delay time calculation section
81 image analysis section
82 image storage section
84 sorting determination section
86 sort signal control section
88 storage section
90 sorting section
100 APD
102 AFE
104 ADC
106 FPGA
108 GbE
110 APD control high-voltage power supply
P particle
SG1 imaging timing instruction signal
D1 image

## Claims

1. An imaging flow cytometer comprising:
a laser unit that emits first laser light and second laser light respectively to a first spot and a second spot on a channel in which a particle flows;
a first imaging section that images the first spot;
a second imaging section that images the second spot;
a first detection device that detects the particle that passes through the first spot;
a second detection device that detects the particle that passes through the second spot;
a first particle detection section that detects the particle based on signals respectively acquired by the first detection device and the second detection device, the first particle detection section issuing, to the first imaging section and the second imaging section, an imaging timing instruction signal that instructs an imaging timing;
a system time management section that issues system times to the first particle detection section;
an image storage section that receives images respectively imaged by the first imaging section and the second imaging section; and
a sorting determination section that determines whether the particle is an objective particle, wherein
the first imaging section and the second imaging section each clip out the image of the particle based on the imaging timing instruction signal.

2. The imaging flow cytometer according to claim 1, wherein the laser unit emits the first laser light and the second laser light being different from each other in wavelength.

3. The imaging flow cytometer according to claim 1 or 2, wherein the first imaging section and the second imaging section each include a line sensor.

4. The imaging flow cytometer according to any of claims 1 to 3, wherein the first imaging section and the second imaging section are disposed along an axial direction of the channel.

5. The imaging flow cytometer according to any of claims 1 to 4, wherein the first particle detection section transmits, to the first imaging section and the second imaging section, an identification number that is given on a particle-by-particle basis.

6. The imaging flow cytometer according to any of claims 1 to 5, wherein the first imaging section and the second imaging section each transmit the clipped image of the particle and the identification number of the particle to the image storage section.

7. The imaging flow cytometer according to any of claims 1 to 6, further comprising:
a delay time calculation section that calculates a delay time of the particle;
a sort signal control section that issues a sort signal for sorting the particle in step with a timing calculated by the delay time calculation section according to the determination of the sorting determination section; and
a sorting section that sorts the objective particle based on the sort signal, wherein
the delay time calculation section calculates the delay time to an arrival of the particle at the sorting section based on the system times corresponding to timings at which the first detection device and the second detection device acquired signals.

8. The imaging flow cytometer according to claim 7, further comprising
a second particle detection section that detects the particle that passes through a detection position positioned downstream from the first spot and the second spot on the channel, wherein
the delay time calculation section compares an estimated arrival time of the particle at the detection position calculated based on system times at which the particle passed through the first spot and the second spot with an arrival time of the particle at the detection position detected by the second particle detection section, and
the sort signal control section adjusts a timing of the sorting section sorting the objective particle based on the comparison between the estimated arrival time and the arrival time.

9. The imaging flow cytometer according to claim 7 or 8, wherein
the detection position is positioned downstream from the sorting section,
the second particle detection section calculates a sort success rate of the particle, and
the delay time calculation section adjusts a timing of the sorting section sorting the objective particle based on the estimated arrival time and the sort success rate.

10. A sorting method comprising:
emitting, by a laser unit, first laser light and second laser light respectively to a first spot and a second spot on a channel in which a particle flows;
detecting, by a first detection device, the particle that passes through the first spot;
detecting, by a second detection device, the particle that passes through the second spot;
imaging the first spot by a first imaging section and imaging the second spot by a second imaging section and clipping out an image of the particle by each of the first imaging section and the second imaging section based on an imaging timing at which the first detection device and the second detection device acquire signals;
determining, by a sorting determination section, whether the particle is an objective particle;
calculating a delay time to an arrival of the particle at a sorting section based on a timing at which the first detection device and the second detection device acquire a signal; and
sorting, by the sorting section, the objective particle based on the delay time of the particle according to the determination of the sorting determination section.

11. The sorting method according to claim 10, further comprising
transmitting, by a first particle detection section, an identification number that is given on a particle-by-particle basis to the first imaging section and the second imaging section, wherein
the first imaging section and the second imaging section each transmit the clipped image of the particle and the identification number of the particle.

12. A calibration method comprising:
emitting, by a laser unit, first laser light and second laser light respectively to a first spot and a second spot on a channel in which a particle flows;
detecting, by a first detection device, the particle that passes through the first spot;
detecting, by a second detection device, the particle that passes through the second spot;
calculating an estimated arrival time of the particle at which the particle passes through a detection position positioned downstream from the first spot and the second spot on the channel based on timings at which the first detection device and the second detection device acquire signals;
detecting the particle that passes through the detection position and detecting an arrival time at which the particle arrives at the detection position, and
adjusting the estimated arrival time based on the arrival time.

13. The calibration method according to claim 12, further comprising
imaging the first spot by a first imaging section and the second spot by a second imaging section and clipping out an image of the particle by each of the first imaging section and the second imaging section based on an imaging timing at which the first detection device and the second detection device acquire signals;
transmitting, by a first particle detection section, an identification number that is given on a particle-by-particle basis to the first imaging section and the second imaging section; and
transmitting, by each of the first imaging section and the second imaging section, the clipped image of the particle and the identification number of the particle.

14. The calibration method according to claim 12 or 13, further comprising adjusting a timing of a sorting section sorting the particle, based on the adjusted estimated arrival time.
